(19) Europäisches Patentamt
European Patent Office
Office européen des brevets

(11) Veröffentlichungsnummer: **0 110 245 B1**

(12) # EUROPÄISCHE PATENTSCHRIFT

(45) Veröffentlichungstag der Patentschrift: **21.08.85**

(51) Int. Cl.⁴: **C 07 C 69/16**, C 07 C 67/00

(21) Anmeldenummer: **83111480.6**

(22) Anmeldetag: **17.11.83**

(54) **Verfahren zur Herstellung von 1,3-Diacetoxy-2-methylenpropan.**

(30) Priorität: **25.11.82 DE 3243545**

(43) Veröffentlichungstag der Anmeldung: **13.06.84 Patentblatt 84/24**

(45) Bekanntmachung des Hinweises auf die Patenterteilung: **21.08.85 Patentblatt 85/34**

(84) Benannte Vertragsstaaten: **CH DE FR GB IT LI SE**

(56) Entgegenhaltungen:
**DE - A - 1 909 964**
**DE - B - 1 931 486**

**JOURNAL OF THE AMERICAN CHEMICAL SOCIETY, Band 78, 22. August 1956 DOUGLAS E. APPLEQUIST et al. "Small-Ring Compounds. XV. Methylenecyclobutene and Related Substances" Seiten 4012-4022**

(73) Patentinhaber: **BAYER AG, Konzernverwaltung RP Patentabteilung, D-5090 Leverkusen 1 Bayerwerk (DE)**

(72) Erfinder: **Jautelat, Manfred, Dr., Müllersbaum 28, D-5093 Burscheid (DE)**
Erfinder: **Arlt, Dieter, Prof. Dr., Rybnikerstrasse 2, D-5000 Köln 80 (DE)**
Erfinder: **Wieland, Helmut, Dr., In den Birken 57, D-5600 Wuppertal 1 (DE)**

**EP 0 110 245 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein chemisch eigenartiges Verfahren zur Herstellung von 1,3-Diacetoxy-2-methylen-propan, das als Zwischenprodukt für die Herstellung von Copolymeren mit wertvollen pharmazeutischen Eigenschaften von Interesse ist (DE-OS 2 705 189).

Es sind bereits mehrere Verfahren zur Herstellung von 1,3-Diacetoxy-2-methylen-propan bekannt geworden. So kann aus Tribrompivalinsäure bei der Umsetzung mit Natriumacetat 1,3-Diacetoxy-2-methylenpropan erhalten werden (Chem. Ber. 91, 938 (1958). Auch die Oxidation von Isobuten oder Metallylacetat mit Sauerstoff in Gegenwart von Essigsäure und Palladium-Katalysatoren führt zu diesem Produkt (DOS 1 909 964, DOS 1 942 014 und DOS 1 957 996). Ferner entsteht bei der Pyrolyse geeigneter Norbornen-Derivate 1,3-Diacetoxy-2-methylenpropan (FR-PS 1 387 099).

Diese Verfahren weisen jedoch einige Nachteile auf. So ist Tribrompivalinsäure nur in mehrstufiger Synthese aus Pentaerythrit mit hohen Kosten erhältlich. Die Oxidationsverfahren und die Pyrolyse erfordern spezielle Apparaturen und Anlagen und kommen deshalb für eine technische Durchführung in üblichen Kesseln nicht in Betracht.

Es wurde gefunden, daß man 1,3-Diacetoxy-2-methylenpropan der Formel

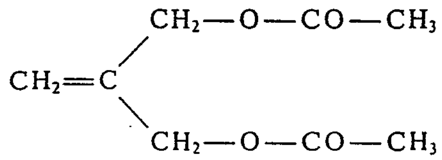

(I)

erhält, wenn man Metallylchlorid der Formel

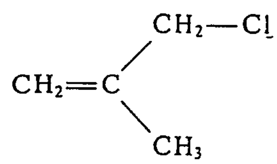

(II)

mit Chlor zum Isomerengemisch der Dichlorisobutene der Formel

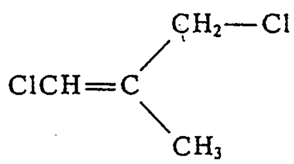

(III)

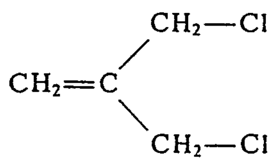

(IV)

umsetzt, dieses Isomerengemisch mit Lewis-Säuren, z. B. Zinkchlorid isomerisiert und dann mit Essigsäure und Basen oder mit Salzen der Essigsäure umsetzt.

Es ist als ausgesprochen überraschend zu bezeichnen, daß das bei der Chlorierung von Methallylchlorid anfallende Isomerengemisch der Dichlorisobutene III und IV durch eine Behandlung mit Lewis-Säuren wie Zinkchlorid in Richtung auf IV verschoben wird.

Das erfindungsgemäße Verfahren weist eine Reihe von Vorteilen auf. So sind die Ausgangsstoffe preiswerte Industrieprodukte. Chlorierung, Isomerisierung und Umsetzung mit Essigsäure können in üblichen Kesseln durchgeführt werden. Das 1,3-Diacetoxy-2-methylenpropan kann durch Destillation einfach gereinigt und isoliert werden.

Der Reaktionsablauf kann durch das folgende Reaktionsschema wiedergegeben werden:

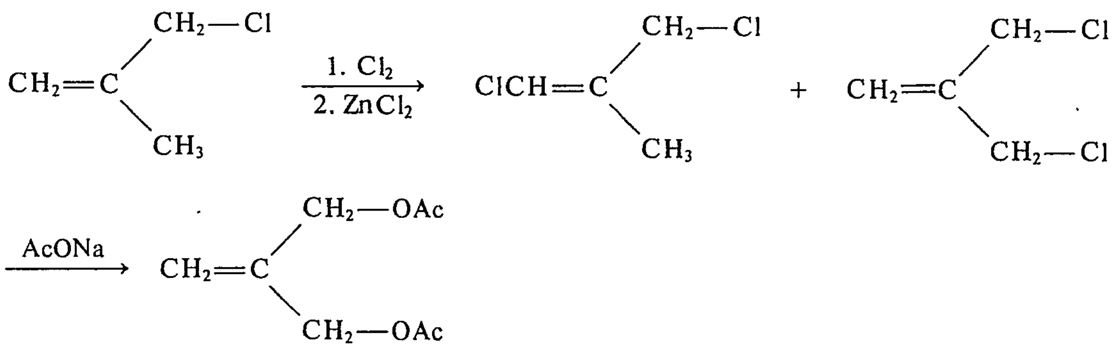


Die Chlorierung von Methallylchlorid ist bekannt (Z. Obsch. Khim. 9, 1261 (1939), JACS 78, 4012 (1956), Tr. po Khim. i Khim. Tekhnol. 1964, 329 u. Ullmann 9, 472 (1975)) und führt zu einem Gemisch, in dem die isomeren Dichlorisobutene III und IV enthalten sind.

Nach dem erfindungsgemäßen Verfahren werden die isomeren Dichlorisobutene III (Kp. 130—132°C) und IV (Kp. 138°C) bei der fraktionierten Destillation gemeinsam von den anderen Produkten abgetrennt. Das Produktverhältnis beträgt ca. 1 : 1. Um eine Trennung dieser Isomeren III und IV zu vermeiden, die einen hohen destillativen Aufwand erfordert, und um das erwünschte Isomere III nutzbar zu machen, wird das Gemisch der Isomeren III und IV einer Isomerisierung mit Lewis-Säuren unterworfen.

Geeignete Lewis-Säuren sind Zinkhalogenide, vorzugsweise Zinkchlorid und Zinkbromid, sowie Bortrifluorid, vorzugsweise Bortrifluoriddiethyletherat.

Die Isomerisierung kann sowohl in Abwesenheit als auch in Gegenwart von inerten Verdünnungsmitteln durchgeführt werden. Als Verdünnungsmittel kommen aliphatische Kohlenwasserstoffe wie Hexan oder Ligroin, chlorierte Kohlenwasserstoffe wie Methylenchlorid oder 1,2-Dichlorethan, Ether wie Diethylether, Tetrahydrofuran oder Dioxan oder Säuren wie Essigsäure in Frage.

Die Isomerisierung kann in einem größeren Temperaturbereich variiert werden. Im allgemeinen arbeitet man zwischen 50 und 180°C, vorzugsweise zwischen 80 und 150°C.

Die Durchführung kann sowohl drucklos im offenen System als auch unter Druck im Autoklaven erfolgen.

Die Menge an Katalysator kann im Bereich von 0,5 bis 20 Mol-% bezogen auf III, vorzugsweise 2 bis 10 Mol-%, variiert werden.

In Abhängigkeit von der Zeitdauer verschiebt sich das Produktverhältnis III/IV von anfänglich ca. 1 : 1 auf 1 : 3.

Die Entfernung des Isomerisierungskatalysators erfolgt entweder durch wäßrige Aufarbeitung der Reaktionsmischung oder durch Vakuumdestillation.

Eine besondere Ausführungsform besteht darin, daß das gesamte Gemisch der Chlorierung von Methallylchlorid ohne vorherige Abtrennung von III und IV isomerisiert und danach erst fraktioniert wird.

Das aus der Isomerisierung erhaltene Produktgemisch aus III und IV, in dem IV angereichert ist, wird entweder fraktioniert und IV alleine in I oder das Gemisch von III und IV in die Acetate I und V übergeführt.

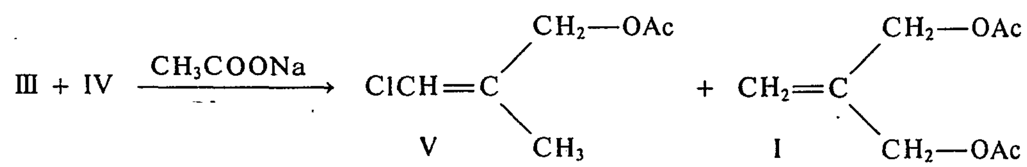


Die Umsetzung wird entweder mit Essigsäure in Gegenwart von Basen wie tert. Aminen, z. B. Triethylamin, Alkalicarbonaten z. B. Kaliumcarbonat oder Alkali- und Erdalkaliacetaten, vorzugsweise Natrium- und Kaliumacetat, in polaren Lösungsmitteln wie Dimethylformamid, Sulfolan, N-Methylpyrrolidon oder Essigsäure ausgeführt. Die Reaktionstemperaturen können in einem größeren Bereich variiert werden. Im allgemeinen arbeitet man zwischen 50 und 200°C, vorzugsweise zwischen 100 und 160°C.

Die Umsetzung kann sowohl unter Druck als auch drucklos ausgeführt werden. Auf ein Mol der Dichlorisobutene III und IV wird 1,5 bis 4 Mol an Base bzw. Acetat, vorzugsweise 2 bis 3 Mol, eingesetzt.

Die Reaktionsmischung wird entweder wäßrig oder nach Filtration durch Destillation aufgearbeitet. Dabei lassen sich die Acetate I und V aufgrund der hohen Siedepunktdifferenz einfach trennen.

Das nach dem erfindungsgemäßen Verfahren hergestellte I dient als Ausgangsprodukt für die

Herstellung der aus DE-OS 2 705 189 bekannten tumorhemmenden Copolymerisate.

Das erfindungsgemäße Verfahren wird durch folgende Beispiele erläutert.

## Beispiel

### a) Chlorierung von Methallylchlorid

181 g (2 Mol) Methallylchlorid werden in Gegenwart von 200 ml verd. Salzsäure bei 20—30° mit 128 g (1,8 Mol) Chlorgas durch Einleiten unter ständigem Rühren chloriert. Die organische Phase wird von der wäßrigen getrennt und enthält laut Gaschromatograph 28,0% 1,3-Dichlor-2-methyl-1-propen III und 39,8% 3-Chlor-2-chlormethyl-1-propen IV. Durch fraktionierte Destillation bei $Kp._{12}$ 29—36°C werden 158 g (1,25 Mol, 63%) Dichlorisobutene III und IV (GC-Gehalt: 43,4% III und 51,8% IV) abgetrennt.

### b) Isomerisierung des Gemisches aus III und IV mit Zinkchlorid

62,5 g (0,5 Mol) Dichlorisobutene im Gemisch aus 49,1% III und 45,8% IV werden mit 6,8 g (0,05 Mol) gepulvertem Zinkchlorid 6 h bei 130°C gerührt. Die direkte Destillation liefert bei $Kp._{12}$ 32° 48,5 g (0,39 Mol, 78%) Produkt, das laut Gaschromatograph 23,9% III und 72,2% IV enthält. Fraktionierung führt zu reinem IV.

### c) Umsetzung von Dichlorisobuten zum Diacetat

12,5 g (0,1 Mol) Dichlorisobuten IV werden in 100 ml Essigsäure gelöst und nach Zutropfen von 22,2 g (0,22 Mol) Triethylamin 10 h unter Rückfluß erhitzt. Man verdünnt mit Methylenchlorid und schüttelt mehrfach mit Wasser aus. Die organ. Phase wird getrocknet und nach dem Abziehen des Lösungsmittels fraktioniert. Bei $Kp._{14}$ 102—106°C werden 16,2 g (0,094 Mol, 94%) 1,3-Diacetoxy-2-methylenpropan isoliert.

**Patentansprüche**

1. Verfahren zur Herstellung von 1,3-Diacetoxy-2-methylen-propan der Formel

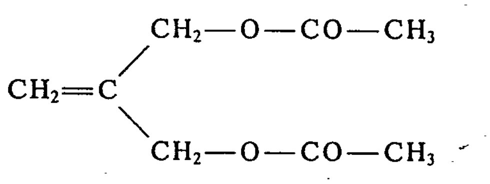

(I)

dadurch gekennzeichnet, daß man Methallylchlorid der Formel

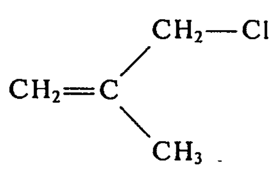

(II)

chloriert, das bei der Chlorierung entstehende Gemisch der isomeren Dichlorisobutene der Formel

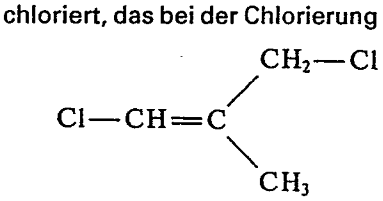

(III)

und

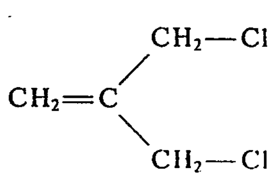

(IV)

mit einer Lewis-Säure isomerisiert und das dabei erhaltene IV mit Essigsäure und Basen oder mit Salzen der Essigsäure umsetzt.

2. Verfahren nach Anspruch 1 dadurch gekennzeichnet, daß man als Lewis-Säure Zinkchlorid oder Zinkbromid verwendet.

3. Verfahren nach Anspruch 1, dadurch gekennzeichnet, daß man als Lewis-Säure Bortrifluoriddiethyletherat verwendet.

## Claims

1. Process for the preparation of 1,3-diacetoxy-2-methylene-propane of the formula

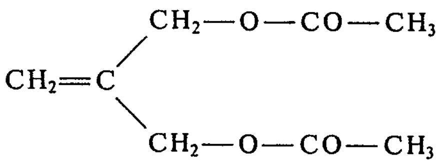

(I)

characterised in that methallyl chloride of the formula

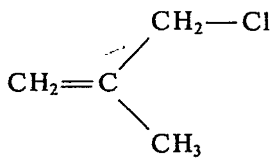

(II)

is chlorinated, the mixture, formed by the chlorination, of the isomeric dichloroisobutenes of the formula

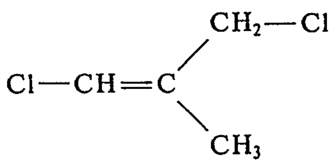

(III)

and

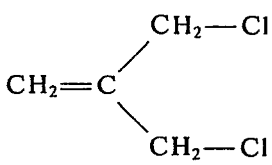

(IV)

is isomerised with a Lewis acid and the IV thereby obtained is reacted with acetic acid and bases or with salts of acetic acid.

2. Process according to Claim 1, characterised in that zinc chloride or zinc bromide is used as the Lewis acid.

3. Process according to Claim 1, characterised in that boron trifluoride diethyl etherate is used as the Lewis acid.

## Revendications

1. Procédé de production de 1,3-diacétoxy-2-méthylènepropane de formule

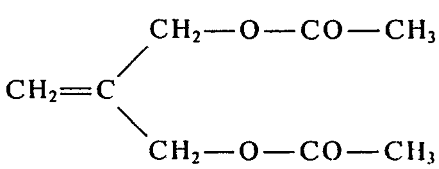

(I)

caractérisé en ce qu'on chlore du chlorure de méthallyle de formule

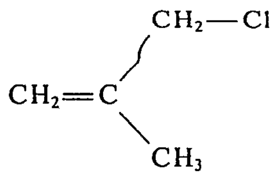

(II)

on isomérise le mélange, formé par chloration, des dichlorisobutènes isomères de formules

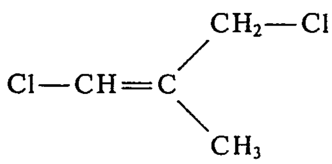

(III)

et

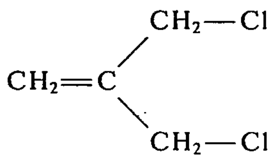

(IV)

avec un acide de Lewis et on fait réagir le composé IV ainisi obtenu avec l'acide acétique et des bases ou avec des sels de l'acide acétique.

2. Procédé suivant la revendication 1, caractérisé en ce qu'on utilise comme acide de Lewis le chlorure de zinc ou le bromure de zinc.

3. Procédé suivant la revendication 1, caractérisé en ce qu'on utilise comme acide de Lewis le complexe d'éther diéthylique du trifluorure de bore.